# EUROPEAN PATENT APPLICATION

(11) **EP 1 543 771 A1**
(43) Date of publication of application: **22.06.2005**
(21) Application number: 03028637.1
(22) Date of filing: 15.12.2003
(51) Int. Cl.: A61B 5/15, A61M 5/32, A61M 25/06

(54) **Flashback blood collection needle with needle shield**

(71) Applicant: BD Medical Products, Pte. Ltd., 639461 Singapore (SG)
(72) Inventor: Alvin Tan Chee Leong, 510225 Singapore (SG); Jon Moh Yaohan, 680678 Singapore (SG)
(74) Representative: Müller-Boré & Partner Patentanwälte

(57) **Abstract**

The needle assembly is provided with the housing (12), an IV cannula (30) for accessing a blood vessel and a non-patient cannula (40) for communication with an evacuated tube. A flashback chamber (26) is provided in the housing at or near the distal end of the housing. A shield (60) is hinged to a portion of the housing proximally of the entrance to the flashback chamber. As a result, good visibility of the flashback chamber is achieved. After use, the shield can be rotated into a closed position for surrounding the IV cannula and avoiding accidental needle sticks.

## Description

### BACKGROUND OF THE INVENTION

### 1. Field of the Invention

A shieldable blood collection needle assembly with a flashback chamber for providing confirmation of venous entry.

### 2. Description of Related Art

Needle assemblies are used for collecting specimens of fluid, such as blood, from a patient. Some such needle assemblies are intended for use with an evacuated tube, such as tubes sold under the trademark VACUTAINER™ by Becton Dickinson. Needle assemblies for use with an evacuated fluid collection tube include a needle hub with a proximal end, a distal end and a passage extending between the ends. The needle assembly further includes at least one needle cannula mounted to the needle hub. The needle cannula includes a sharply pointed distal end that projects distally beyond the needle hub, a proximal end that projects proximally beyond the needle hub and a lumen that provides communication between the opposed ends of the needle cannula. Some needle assemblies include separate proximal and distal cannulas and rely upon a portion of the needle hub to provide communication between the lumens of the respective cannulas. The distal end of the needle cannula typically is beveled to a tip that is sufficiently sharp for piercing the skin of the patient and accessing the vein or other source of fluid that is to be collected. The proximal end of the needle cannula is configured for piercing the rubber stopper on an evacuated tube. The proximal end of the needle cannula typically is covered by a needle pierceable resealable multi-sample sleeve. The sleeve is compressed by the rubber stopper of the evacuated tube and punctured by the proximal end of the needle cannula as the proximal end of the needle cannula is urged into communication with the evacuated tube.

One problem with collecting fluid from a patient relates to uncertainty during attempts to access the proper source of fluid in the patient. For example, a blood collection procedure typically requires the phlebotomist to visually locate a vein and then to enter the vein with the distal end of the needle cannula. The phlebotomist may not have a positive indication of venous entry at this stage. An evacuated tube then is placed in communication with the proximal end of the needle cannula once the phlebotomist is reasonably certain that the targeted blood vessel has been entered. The low pressure within the evacuated tube then cooperates with the higher pressure of the blood to generate a flow of blood into the tube. The flow of blood into the tube may be the first positive indication to the phlebotomist that the targeted blood vessel has been accessed. The initial flow of blood along the long needle cannula and into the evacuated tube may take a relatively long time based on several factors, including the relative pressure levels and the length and cross-sectional dimensions of the needle cannula. The phlebotomist may interpret the absence of an immediate flow of blood as being a sign that the blood vessel was not targeted properly, when in fact the absence of a visible blood flow in the evacuated tube may be attributable to pressure and fluid flow characteristics. Thus, the phlebotomist may unnecessarily withdraw the needle and start the blood collection procedure again. As a result, time is wasted and trauma for the patient is increased. In view of the above problems, many fluid collection needle assemblies are provided with a flashback chamber that communicates with the needle cannula. The flashback chamber typically is formed at least partly from a transparent or translucent material and is intended to receive a portion of the blood flow shortly after a vein has been accessed properly.

The blood collection needle assembly is withdrawn from the patient after a suitable number of samples have been collected. The used needle assembly then is discarded. The medical profession is well aware that accidental sticks with a used needle cannula can transmit disease. Accordingly, it is desirable to shield the used needle cannula immediately after the needle cannula has been withdrawn from the patient. Shields have taken many different forms. For example, some shields telescope in a distal to proximal direction over the needle cannula and frictionally engage the needle hub. Shields of this type create the risk of an accidental needle stick during shielding if the shield is misaligned with the needle cannula. Some shields of this general type are provided with enlarged collars that are intended to minimize the risk of accidental sticks during shielding. However, shields of this general type are not preferred. Other shields are telescoped over the needle hub and can be moved distally over the needle cannula to effect shielding. Shields of this general type are safe and effective and are used in many situations. However, shields of this type can interfere with the normal usage of some medical implements. Other shields are hingedly mounted to or near the needle hub and can be rotated from an open position where the needle cannula is exposed to a closed position where the needle cannula is shielded. Shields of this type also work very well and are widely accepted. However, the existence of a hinged shield on a fluid collection needle assembly with a flashback chamber is intuitively problematic in view of the complexities of providing both shielding and an unobstructed view of the flashback chamber.

### SUMMARY OF THE INVENTION

The subject invention is directed to a shieldable fluid collection needle assembly. The needle assembly includes a housing with a proximal end wall, a distal end wall and an external sidewall extending between the proximal and distal end walls such that a chamber is defined between the end walls and the external sidewall. The external sidewall may be formed unitarily with at least one of the end walls. Additionally, at least a portion of the external sidewall is formed from a transparent or translucent material so that the interior of the chamber within the housing is visible. A proximal passage extends through the proximal end wall of the housing and a distal passage extends through the distal end wall of the housing. The proximal and distal passages maybe axially aligned with one another. The housing may further include an internal sidewall extending proximally from the distal end wall and surrounding the entry of the distal passage into the chamber. The internal sidewall also is a transparent or translucent material.

The needle assembly further includes a distal cannula mounted in the distal passage of the housing and projecting distally beyond the housing. The distal cannula includes a distal end spaced externally from the housing. The distal end may be beveled to a tip that is sufficiently sharp to pierce skin of a patient. The distal cannula includes a lumen that extends from the distal end of the distal cannula and communicates with the chamber of the housing.

The needle assembly further includes a proximal cannula mounted in the proximal passage of the housing. The proximal cannula includes a proximal end that projects proximally beyond the housing. Additionally, the proximal cannula includes a lumen that extends from the proximal end and into communication with the chamber. The proximal cannula also includes a distal end that may extend into the chamber so that the proximal end of the distal cannula and the distal end of the proximal cannula substantially align with one another and are slightly spaced from one another. In an alternate embodiment, distal and proximal cannula may be integral with one another and may include a transverse slot or aperture to provide communication between the lumens of the distal and proximal cannulas with the chamber in the housing.

The proximal end of the distal cannula preferably is at or near the distal end wall of the housing. Additionally, the distal end of the proximal cannula preferably extends through a major portion of the chamber of the housing and to a location near the distal end wall of the housing. Thus, both the distal and proximal cannulas preferably communicate with the chamber of the needle assembly at a location near the distal end wall of the housing. As a result, the first indication of fluid flow through the distal cannula will occur at a location at or substantially adjacent the distal end wall of the housing. In a preferred embodiment, the housing may have the internal sidewall described above and the distal end of the proximal cannula may project axially into the cylindrical space bounded by the internal sidewall.

The needle assembly of the subject invention further includes a shield that may be hinged to a location on the housing proximally of the location where the first indication of flashback occurs. The shield initially is in an open position where the shield is spaced from the distal cannula and the chamber. Hence, the shield does not impede usage of the distal cannula or visual observation of the chamber when the shield is in its open position. However, the shield can be rotated from the open position to a closed position where the shield surrounds the distal cannula. The shield and/or the housing may include at least one locking element for locking the shield in the closed position. The locking means can include a resiliently deflectable cannula lock configured for engaging the needle cannula when the shield is in the closed position. The shield may further include locks for engaging structure on the housing when the shield is in the closed position. The engagement between structure on the shield and structure on the housing may function to lock the shield in the closed position. Alternatively, primary locking of the shield in the closed position may be achieved by the cannula lock, and the engagement between the shield and the housing may be provided primarily for audible and tactile indication of complete shielding.

### DESCRIPTION OF THE DRAWINGS

FIG. 1 is an exploded perspective view of a needle assembly in accordance with the invention.

FIG. 2 is a longitudinal cross-sectional view of the needle assembly prior to use.

FIG. 3 is a side elevational view of the needle assembly after being placed in communication with a blood vessel, but before being placed in communication with an evacuated tube.

FIG. 4 is a longitudinal cross-sectional view of the needle assembly during use and after being placed in communication with an evacuated tube.

FIG. 5 is a side elevational view of the needle assembly after complete shielding.

### DETAILED DESCRIPTION

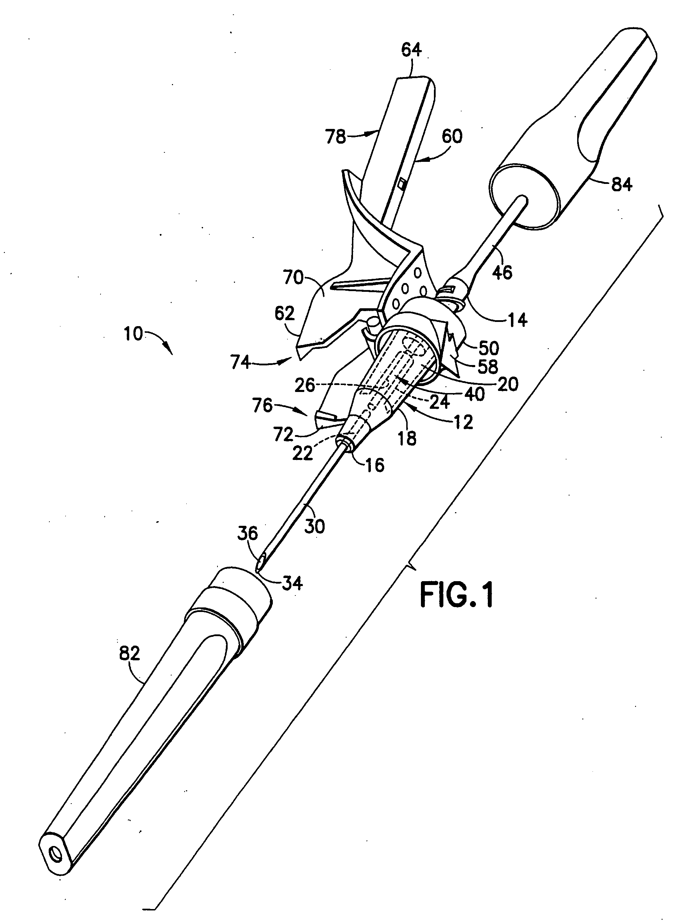

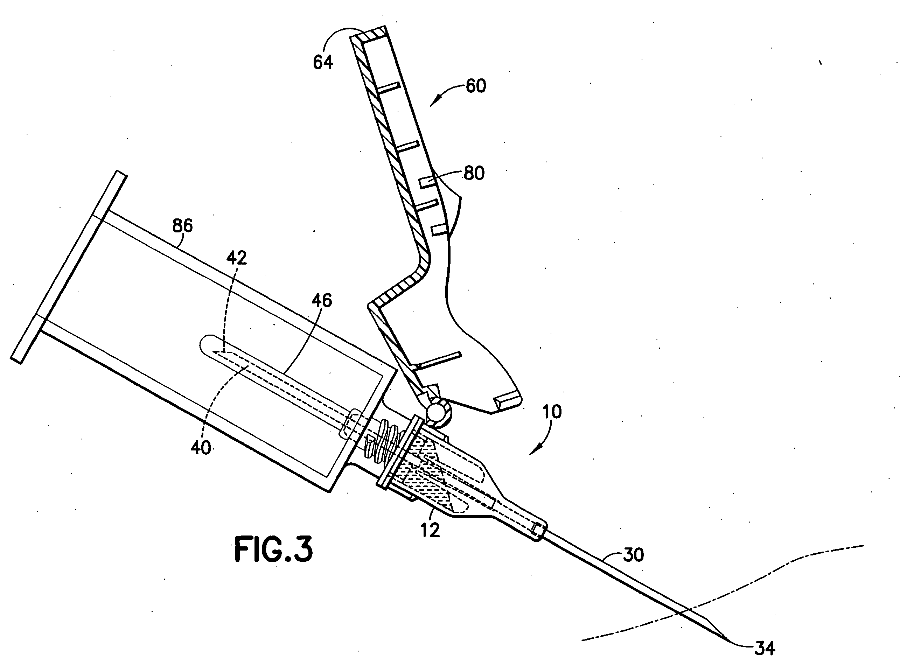

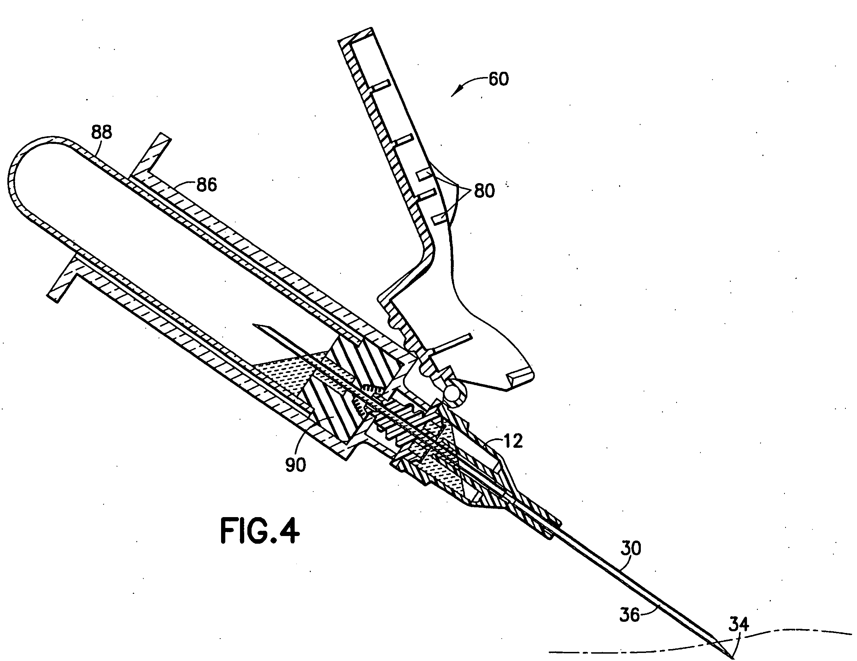

A needle assembly in accordance with the subject invention is identified generally by the numeral **10** in FIGS. 1-5. Needle assembly **10** includes a housing **12** with a proximal end **14**, a distal end **16** and a generally frustum-shaped outer sidewall **18** extending between the ends. Outer sidewall **18** is formed from a transparent or translucent plastic material and defines a chamber **20** within housing **12** between proximal end **14** and distal end **16**. A proximal passage (not shown) extends through proximal end **14** of housing **12** and communicates with chamber **20**. A distal passage **22** extends through distal end **16** of housing **12** and also communicates with chamber **20**. Housing **12** further includes a cylindrical inner sidewall **24** that extends from distal end **16** toward proximal end **14**. Inner sidewall **24** is substantially concentrically disposed within outer sidewall **18** and is substantially concentrically generated about distal passage **22**. Inner sidewall **24** also is formed from a transparent or translucent plastic material. Hence, a fluid material in a portion of chamber **20** bounded by inner sidewall **24** is readily visible from locations externally of outer sidewall **18**. Thus, inner sidewall **24** in this embodiment defines a flashback chamber **26**, and fluid collected in flashback chamber **26** can be observed from locations externally of needle assembly **10.** Chamber **26** communicates with chamber **20**, and hence chamber **20** may also contribute to an indication of flashback.

Needle assembly **10** further includes an IV cannula **30** with a proximal end **32,** a distal end **34** and a lumen **36** extending between the ends. Distal end **34** of IV cannula **30** is disposed externally of housing **12** and is beveled to a sufficiently sharp tip for piercing skin and tissue of a patient. IV cannula **30** is mounted in distal passage **22** of housing **12** so that proximal end **32** of IV cannula **30** is substantially adjacent distal end **16** of housing **12**. Thus, lumen **36** through IV cannula **30** communicates with flashback chamber **26** at a location substantially adjacent distal end **16** of housing **12**.

Needle assembly **10** further includes a non-patient (NP) cannula **40** mounted in the proximal passage at proximal end **14** of housing **12**. NP cannula **40** includes a proximal end **42** disposed externally of housing **12,** a distal end **44** disposed in flashback chamber **26** and a lumen (not shown) extending between the ends. The proximal end **42** is beveled to a sufficiently sharp tip for piercing a rubber stopper of an evacuated tube as explained further herein. The lumen through NP cannula **40** is aligned substantially axially with lumen **36** through IV cannula **30**. Distal end **44** of NP cannula **40** preferably is spaced only a short distance from proximal end **32** of IV cannula **30**, such as 0.5 mm - 1.2 mm.

Needle assembly **10** further includes a sleeve **46** mounted over portions of NP cannula **40** that are disposed externally of housing **12**. Sleeve **46** is mounted to proximal end **14** of housing **12**, and is formed from a material that is substantially impervious to liquid and fluid. However, sleeve **46** also is formed from a material that is readily pierceable by proximal end **42** of NP cannula **40** and that is resiliently resealable. Thus, the rubber stopper of an evacuated tube can be urged against sleeve **46** and will cause sleeve **46** to collapse distally. As a result, the beveled tip at proximal end **42** of NP cannula **40** will pierce through sleeve **46** and through the rubber stopper on the evacuated tube.

IV cannula **30**, NP cannula **40** and housing **12** cooperate to provide an early indication of venous entry due to the flashback of blood in chamber **26** and/or chamber **20** of housing **12**. However, other flashback designs can be incorporated into the needle assembly of the subject invention. These other designs include arrangements were blood first fills the equivalent of sleeve **46** on needle assembly **10** and then flows into a flashback chamber. Still other designs include the use of a vented plug. Flashback chamber designs that can be incorporated into the subject invention are shown, for example, in U.S. Patent No. 5,542,932, U.S. Patent No. 5,824,001 and others.

Needle assembly **10** further includes a collar **50** securely mounted around proximal portions of housing **12**. Collar **50** may be secured mechanically in position on housing **12**, may be bonded to housing **12** or may be molded as part of housing **12**. Collar **50** includes a proximal end **52** substantially aligned with proximal portions of chamber **20** and a distal end **54** disposed proximally of proximal end **32** of IV cannula **30** and near proximal end of inner sidewall **24.** Accordingly, collar **50** will not impair the ability to observe regions of flashback chamber **26** adjacent proximal end **32** of IV cannula **30**. Distal end **54** of collar **50** is provided with a C-shaped hook **56** generated about an axis that extends transverse to IV cannula **30** and NP cannula **40**. Collar **50** further includes a chevron-shaped projection **58** at a location diametrically opposite hook **56**. Collar **50** preferably is secured to housing **12** such that a plane passing symmetrically through C-shaped hook **56** and through the diametrically opposite chevron-shaped projection **58** also passes symmetrically through the bevel at distal end **34** of IV cannula **30**. However, in other embodiments collar **50** may be rotatably mounted to housing **12** so that the orientation of the plane passing symmetrically through hook **56** and through projection **58** can be varied relative to a plane passing symmetrically through the bevel at distal end **34** of IV cannula **30**.

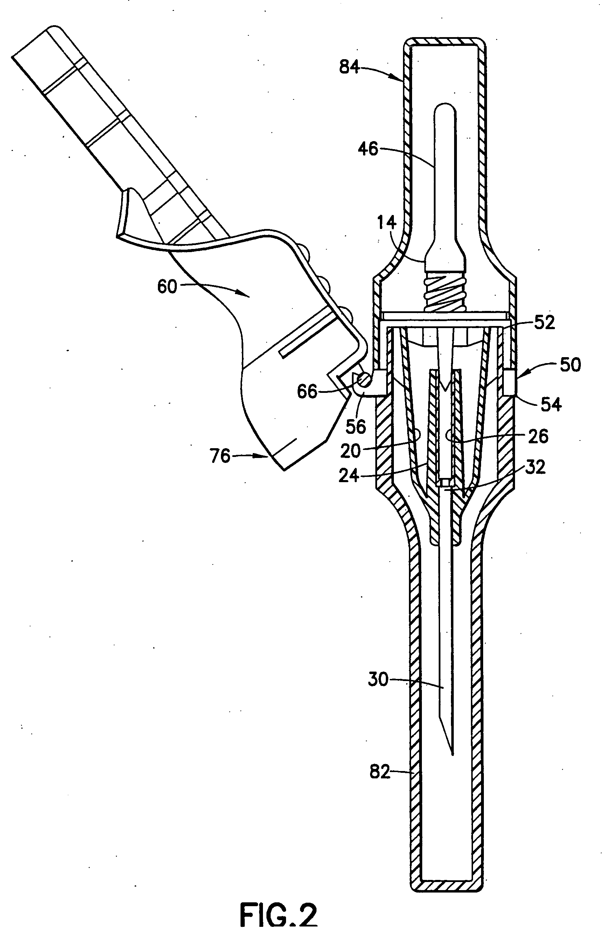

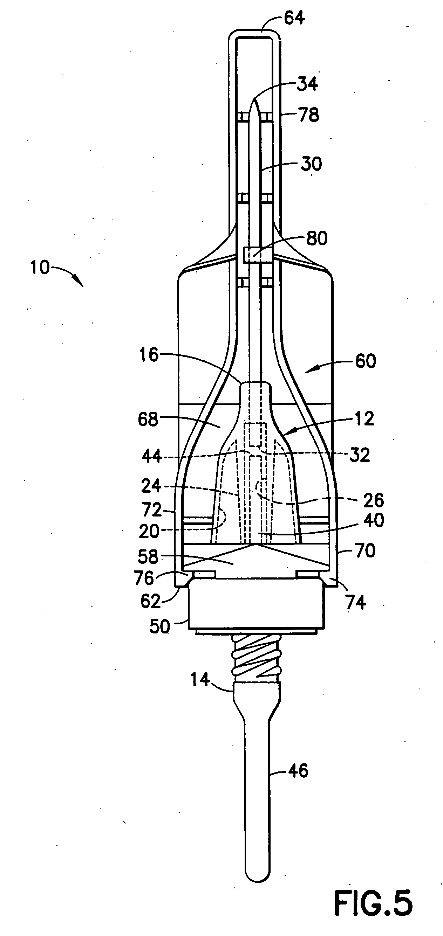

Needle assembly **10** further includes a shield **60** with a proximal end **62** and a distal end **64**. Portions of shield **60** near proximal end **62** include a hinge pin **66** that is snapped into engagement with hook **56** so that shield **60** can rotate relative to hook **56** from an open position shown in FIGS. 1 and 2 to a closed position shown in FIG. 5. In the open position, shield **60** is spaced from IV cannula **30** and is aligned to IV cannula **30** at an obtuse angle of about 120°. The preferred orientation of collar **50** relative to the bevel at distal end **34** of IV cannula **30** provides a clear indication of the preferred bevel-up orientation of IV cannula **30** that is employed by most health care technicians during use. Additionally, the orientation of collar **50** on housing **12** ensure that shield **60** will not interfere with the view of IV cannula **30** during insertion. The above-referenced option of a rotatable collar enables the user to select a preferred orientation of shield **60** relative to the bevel at distal end **34** of IV cannula **30** and relative to the patient and other medical equipment that may be placed in communication with the patient. Portions of shield **60** near proximal end **62** further include a top wall **68** and sidewalls **70** and **72** that project from top wall **68**. Portions of sidewalls **70** and **72** at proximal end **62** of shield **60** are spaced apart by a distance that equals or exceeds the width of outer sidewall **18** of housing **12**. Thus, sidewalls **70** and **72** easily can be rotated on opposite respective sides of outer sidewall **18** as shield **60** is rotated within hook **56**. The wide spacing of sidewalls **70** and **72** also facilitates observations of the flashback chamber **26**. Opposed facing surfaces of sidewalls **70** and **72** at proximal end **62** of shield **60** are formed with collar catches **74** and **76** respectively. Collar catches **74** and **76** are configured to snap into engagement with chevron-shaped latch **58** on collar **50**. Top wall **68** is provided with an array of tactile bumps on the surface facing away from sidewalls **70** and **72** to provide a visual indication of an actuation landing for a thumb or forefinger and to enhance frictional engagement by the thumb or forefinger when rotating shield **60**.

Shield **60** narrows at locations between top wall **68** and distal end **64** and defines a substantially U-shaped channel of sufficient width to receive IV cannula **30**. As shown most clearly in FIG. 5, channel **78** is sufficiently long to encompass all of IV needle **30**. Interior portions of channel **78** include a resiliently deflectable cannula lock **80** that is angled from one sidewall of channel **78** toward the opposed sidewall and toward the top wall. Cannula lock **80** will yield in response to contact with IV cannula **30** as shield **60** is rotated toward IV cannula **30**. Cannula lock **80** is further dimensioned to move passed IV cannula **30** when shield **60** approaches the closed position. Hence, cannula lock **80** will return resiliently toward an undeflected position and will trap IV cannula **30** within shield **60**. Although the figures herein depict a single cannula lock **80**, plural cannula locks may be provided. Additionally, cannula locks may extend from each of the two sidewalls of U-shaped channel **80**. Other structures for locking IV cannula **30** in shield **60** can be provided. These include other configurations for plastic or metal cannula locks that deflect in response to contact with IV cannula **30** and then resiliently move to trap IV cannula **30**. Alternatively, the cannula lock may require some form of user activation, such as rotation or axial movement of a portion of the shield.

Needle assembly **10** further include an IV shield **82** and a NP shield **84** mounted respectively over IV cannula **30** and NP cannula **40** and frictionally retained on collar **50.** Shields **82** and **84** can be separated by an appropriate application of pulling and/or slight twisting force.

Needle assembly **10** is used by first separating NP shield **84** from collar **50** and then threading distal end **14** of housing **12** into a needle holder **86**. Shield **60** then is rotated into the fully open position shown in FIGS. 1 and 2 where shield **60** is spaced from IV cannula **30** and is aligned to IV cannula **30** at an obtuse angle of about 120°. IV shield **78** then is removed from its engagement with collar **50.** The phlebotomist then guides distal end **34** of IV cannula **30** into a targeted blood vessel. A pressure differential between the blood in the vein and the pressure within housing **12** will cause blood to flow through lumen **36** of IV cannula **30**. Blood will appear in flashback chamber **26** very quickly after access to the blood vessel has been attained. This blood can be seen in flashback chamber **26** by the phlebotomist due to the disposition and alignment of shield **60** in the open position. The phlebotomist then inserts an evacuated tube **88** into needle holder **82**. Evacuated tube **88** includes a rubber stopper **90** that collapses sleeve **46** distally. As a result, proximal end **42** of NP cannula **40** pierces rubber sleeve **46** and then pierces stopper **90** of evacuated tube **88**. The phlebotomist accumulates one or more samples of blood in this manner.

After collecting the last sample of blood, the phlebotomist urges needle assembly **10** and needle holder **86** from the patient and immediately exerts pressure with a thumb or forefinger on top wall **68** of shield **60**. As a result, shield **60** rotates about hook **56** into a closed position, as shown in FIG. 5, so that IV cannula **30** is surrounded by channel **78** of shield **60**. Sufficient rotation causes cannula lock **80** to engage IV cannula **30** and to deflect. Further rotation of shield **60** causes cannula lock **80** to pass IV cannula **30**. As a result, cannula lock **80** resiliently returns toward and undeflected condition and traps IV cannula **30**. Simultaneously, collar catches **74** and **76** snap into engagement with chevron-shaped latch **58** on collar **50** to provide an audible and tactile indication of shielding. The safely shielded needle assembly then can be discarded in a sharps receptacle.

The needle assembly provides effective shielding without impeding use of needle assembly **10** and without obscuring observation of flashback chamber **26**. The high visibility of flashback chamber **26** is partly attributable to the extreme distal position of the space between IV cannula **30** and NP cannula **40** and the corresponding proximal position of shield **60** in the open position. Additionally, as shown most clearly in FIG. 1, proximal end **62** of shield **60** is widely open between sidewalls **70** and **72** to provide a broad range of acceptable viewing angles.

The above-described needle assembly has hinge pin **66** of shield **60** snapped into engagement with C-shaped hook **56** on collar **50** for hinged rotation of shield **60** relative to collar **50** and relative to housing **12**. However, other structures for accommodating hinged movement of shield **60** relative to collar **50** and/or housing **12** can be provided. These include forming shield **60** unitarily with collar **50** so that a living hinged connection between shield **60** and collar **50** is provided. A shield with a living hinge is shown, for example, in U.S. Patent No. 5,681,295. Such hinged connections can also include an over-center hinge where the shield is substantially unbiased in the fully open position and in the fully closed position. However, the user may have to overcome a bias in the over-center hinge during the initial phases of rotation from the open position to the closed position. The over-center hinge then resiliently returns toward an unbiased condition and accelerates the hinge towards the closed position.

## Claims

1. A needle assembly comprising: a housing having proximal and distal ends and a chamber wall extending between said ends for defining a chamber in said housing, at least a portion of said chamber wall being formed from a material that permits observation of a fluid flowing into said chamber, and an IV cannula projecting distally from said housing and communicating with said chamber, a shield hinged to said housing and being positionable in an open position where said shield is spaced from said IV cannula and aligned to permit observation of said chamber, said shield further being rotatable to a closed position where said shield covers said IV cannula.

2. The needle assembly of Claim 1, wherein at least a portion of said chamber is disposed distally of a location at which said shield is hingedly connected to said housing.

3. The needle assembly of Claim 2, wherein said IV cannula communicates with a portion of said chamber adjacent said distal end of said housing, said shield being hingedly connected to a portion of said housing substantially adjacent said proximal end of said housing.

4. The needle assembly of any of Claims 1-3, wherein said shield includes at least one lock for locked engagement with said IV cannula when said shield is in said closed position.

5. The needle assembly of any of Claims 1-4, wherein said shield comprises at least one catch for engaging a portion of said housing spaced from said hinged connection when said shield is in said closed position.

6. The needle assembly of Claim 5, wherein said at least one catch on said shield for engaging said housing is configured for generating audible and tactile indications of shielding.

7. The needle assembly of any of Claims 1-6, further comprising a non-patient cannula projecting from said housing and communicating with said chamber.

8. The needle assembly of Claim 7, wherein said non-patient cannula includes a distal end disposed in said chamber, said distal end of said non-patient cannula being disposed distally of the hinged connection of said shield to said housing.

9. The needle assembly of Claim 8, wherein said non-patient cannula extends sufficiently through said housing such that said distal end of said non-patient cannula is substantially adjacent said distal end of said housing, said distal end of said non-patient cannula being substantially aligned with and spaced slightly from said IV cannula.

10. The needle assembly of any of Claims 1-9, wherein said chamber wall is an outer chamber wall, said housing further comprising an inner chamber wall defining a flashback chamber within said housing, said inner chamber wall extending from said distal end of said housing toward said proximal end of said housing such that said IV cannula communicates with said flashback chamber defined by said inner chamber wall.

11. The needle assembly of any of Claims 1-10, wherein said housing comprises a collar mounted around a portion of said housing, said shield being hinged to said collar.

12. The needle assembly of Claim 11, wherein said collar is fixed to said housing.

13. The needle assembly of Claim 11, wherein said housing includes a central axis extending between said proximal and distal ends, said collar being rotatable around said central axis for orienting said shield at a preferred rotational alignment relative to said IV cannula.

14. The needle assembly of any of Claims 11-13, wherein said collar is mounted to said housing at a location proximally of said chamber.
